# EUROPEAN PATENT SPECIFICATION

(11) **EP 4 276 109 B1**
(45) Date of publication and mention of the grant of the patent: **04.02.2026**
(21) Application number: 21917631.0
(22) Date of filing: 13.12.2021
(51) Int. Cl.: C07K 16/28, C12N 15/12, G01N 33/50, G01N 33/53, G01N 33/68, C07K 14/705

(54) **BIOMARKER FOR DETERMINING FERTILITY, AND DETERMINING METHOD USING SAME**
BIOMARKER ZUR BESTIMMUNG DER FRUCHTBARKEIT UND BESTIMMUNGSVERFAHREN DAMIT
BIOMARQUEUR POUR DÉTERMINER LA FERTILITÉ ET MÉTHODE DE DÉTERMINATION L'UTILISANT

(30) Priority: 05.01.2021 JP 2021000399; 30.08.2021 JP 2021139495
(43) Date of publication of application: 15.11.2023
(73) Proprietor: Public University Corporation Yokohama City University, Yokohama-shi, Kanagawa 236-0027 (JP); Tosoh Corporation, Yamaguchi 746-8501 (JP)
(72) Inventor: MIYAGI, Etsuko, Yokohama-shi, Kanagawa 236-0004 (JP); MURASE, Mariko, Yokohama-shi, Kanagawa 232-0024 (JP); HAYAMA, Tomonari, Yokohama-shi, Kanagawa 232-0024 (JP); RYO, Akihide, Yokohama-shi, Kanagawa 236-0004 (JP); HORIUCHI, Yayoi, Yokohama-shi, Kanagawa 236-0004 (JP); KOBORI, Hiroki, Ayase-shi, Kanagawa 252-1123 (JP); OHTAKE, Norihisa, Ayase-shi, Kanagawa 252-1123 (JP)
(74) Representative: Novagraaf International SA
(86) International application number: PCT/JP2021/045799
(87) International publication number: WO 2022/149410

(56) References cited:
- CN-A- 111 579 798
- JP-A- 2010 515 909
- JP-A- 2013 510 575
- JP-A- 2016 173 366
- JP-A- 2017 158 579
- JP-A- 2017 510 250
- JP-A- 2018 513 979
- US-A1- 2002 155 995
- IDA VOGEL ET AL: "Preterm delivery predicted by soluble CD163 and CRP in women with symptoms of preterm delivery", BJOG: AN INTERNATIONAL JOURNAL OF OBSTETRICS AND GYNAECOLOGY, WILEY-BLACKWELL PUBLISHING LTD, GB, vol. 112, no. 6, 25 January 2005 (2005-01-25), pages 737 - 742, XP072234582, ISSN: 1470-0328, DOI: 10.1111/J.1471-0528.2005.00557.X
- FAN XIANGXIU ET AL: "Endometrial CD138 count appears to be a negative prognostic indicator for patients who have experienced previous embryo transfer failure", FERTILITY AND STERILITY, ELSEVIER, AMSTERDAM, NL, vol. 112, no. 6, 1 December 2019 (2019-12-01), pages 1103 - 1111, XP085949894, ISSN: 0015-0282, [retrieved on 20191213], DOI: 10.1016/J.FERTNSTERT.2019.08.006
- ANONYMOUS: "Human Soluble CD163 (sCD163) Assay Kit", FUNAKOSHI CO., LTD., 22 May 2006 (2006-05-22), XP055949790, Retrieved from the Internet <URL:www.funakoshi.co.jp/contents/561>
- VOGEL IDA, ET AL.: "Preterm delivery predicted by soluble CD163 and CRP in women with symptoms of preterm delivery", BJOG, vol. 112, no. 6, 30 June 2005 (2005-06-30), pages 737 - 742, XP055949794, DOI: 10.1111/j.1471-0528.2005.00557.x
- DIAO LIANGHUI, CAI SONGCHEN, HUANG CHUNYU, LI LONGFEI, YU SHUYI, WANG LINLIN, LIU SU, LI YUYE, ZENG YONG: "New endometrial immune cell-based score (EI-score) for the prediction of implantation success for patients undergoing IVF/ICSI", PLACENTA, W.B. SAUNDERS, GB, vol. 99, 1 September 2020 (2020-09-01), GB , pages 180 - 188, XP055949795, ISSN: 0143-4004, DOI: 10.1016/j.placenta.2020.07.025
- YIN, YONG-XIANG: "Expression of CD68 and CD163 in unexplained infertility endometrial tissue", GUANGDONG YIXUE, vol. 35, no. 10, 1 January 2014 (2014-01-01), CN , pages 1600 - 1602, XP009538197, ISSN: 1001-9448
- SHARPS MEGAN C., ET AL.: "Increased placental macrophages and a pro-inflammatory profile in placentas and maternal serum in infants with a decreased growth rate in the third trimester of pregnancy", AMERICAN JOURNAL OF REPRODUCTIVE IMMUNOLOGY, vol. 84, no. 3, 31 May 2000 (2000-05-31), pages e13267, XP055949799, DOI: 10.1111/aji.13267
- TANAKA, AYAKA: "O-154 Probucol results with Scavenger receptor class B type I (SR‐BI) deficient apolipoprotein E (ApoE) low gene mutations in mouse fertility", JOURNAL OF JAPAN SOCIETY FOR REPRODUCTIVE MEDICINE, JAPAN SOCIETY FOR REPRODUCTIVE MEDICINE, JP, vol. 62, no. 4, 1 October 2017 (2017-10-01), JP , pages 216 (378), XP009538321, ISSN: 1881-0098
- A. KOLMAKOVA, J. WANG, R. BROGAN, C. CHAFFIN, A. RODRIGUEZ: "Deficiency of Scavenger Receptor Class B Type I Negatively Affects Progesterone Secretion in Human Granulosa Cells", ENDOCRINOLOGY, ASSOCIATION FOR THE STUDY OF INTERNAL SECRETIONS, vol. 151, no. 11, 1 November 2010 (2010-11-01), pages 5519 - 5527, XP055090573, ISSN: 00137227, DOI: 10.1210/en.2010-0347
- XU JERRY Z, KUMAR RAMESH, GONG HAOLI, LIU LUYAO, RAMOS-SOLIS NICOLE, LI YUJING, DERBIGNY WILBERT A, XU CITATION, JZ, KUMAR R, G: "Toll-Like Receptor 3 Deficiency Leads to Altered Immune Responses to Chlamydia trachomatis Infection in Human Oviduct Epithelial Cells", INFECTION AND IMMUNITY, vol. 87, no. 10, 31 October 2019 (2019-10-31), pages e00483 - 19, XP055949804, DOI: 10.1128/IAI.00483-19
- FAN XIANGXIU; YANG YUANYUAN; WEN QUAN; LI YUAN; MENG FEI; LIAO JINGNAN; CHEN HUIJUN; LU GUANG-XIU; LIN GE; GONG FEI: "CD19 and intraglandular CD163-positivity as prognostic indicators of pregnancy outcome in CD138-negative women with a previous fresh-embryo-transfer failure", JOURNAL OF REPRODUCTIVE IMMUNOLOGY, ELSEVIER SCIENCE IRELAND LTD., IE, vol. 147, 26 August 2021 (2021-08-26), IE , XP086783545, ISSN: 0165-0378, DOI: 10.1016/j.jri.2021.103362
- TAVARES PEREIRA MIGUEL, NOWACZYK RENATA, PAYAN-CARREIRA RITA, MIRANDA SONIA, ASLAN SELIM, KAYA DUYGU, KOWALEWSKI MARIUSZ P.: "Selected Uterine Immune Events Associated With the Establishment of Pregnancy in the Dog", FRONTIERS IN VETERINARY SCIENCE, vol. 7, 1 January 2021 (2021-01-01), pages 625921, XP055949805, DOI: 10.3389/fvets.2020.625921

## Description

### TECHNICAL FIELD

The present invention relates to a method of determining fertility of an ovum or embryo using as an index the soluble CD163 concentration in a human body fluid.

### BACKGROUND ART

In Japan, the number of treatment cycles in infertility treatment in 2017 exceeded 400,000, and the number of births exceeded 50,000. It has been predicted that the trend toward delayed marriage and the like may still lead to an increase the number of cases of infertility treatment and an increase in the number of births by in vitro fertilization (Non-Patent Document 1).

Factors that may cause infertility are present in both males and females. Examples of such factors in males include spermatogenic dysfunction, obstruction of sperm transport, and sperm dysfunction. Examples of such factors in females include aging, gynecological diseases (such as endometriosis and uterine fibroids), endocrine diseases, and metabolic diseases. However, there are also cases whose causes are uncertain and which show no abnormality in common infertility tests, and, in cases where no obvious abnormality is found, the low infertility may be due to deterioration of ovum quality. The fertility herein means ease of pregnancy, and means the level of the ability of an ovum, or an embryo that develops from an ovum after fertilization, to achieve the entire process from implantation to pregnancy. Since the determination of the quality of ova obtained by ovum pick up is impossible, the probability of achievement of the implantation is at most about 30% even in cases where in vitro fertilization is carried out followed by transfer of a favorable embryo obtained through embryo culture (Non-Patent Document 1). In order to increase the implantation rate, attempts have been made by transferring a plurality of embryos obtained by in vitro fertilization. However, since multiple pregnancy places a heavy physical burden, it has currently been proposed that single embryo transfer, in which only one embryo is transferred, should be carried out (Non-Patent Document 2).

The current embryo transfer includes cleavage-stage embryo transfer, which is carried out with a cleavage-stage embryo, and blastocyst transfer, in which an embryo that has become a blastocyst is transferred. As morphological methods for the evaluation in these embryo transfers, Veeck classification (Non-Patent Document 3) and Gardner classification (Non-Patent Document 4), respectively, are mainly employed. In general, the embryos obtained by in vitro fertilization are subjected to morphological evaluation, and then the top-quality embryo in the same cycle is transferred. However, the transfer of the top-quality embryo does not necessarily lead to implantation and pregnancy, and the opposite may also occur. Thus, the morphological evaluation of embryos does not strictly correlate with the actual qualities of the embryos. In recent years, time-lapse culture, in which the developmental process of an embryo is observed, has been developed. Although a method in which evaluation is carried out based on the developmental process of the embryo rather than the final morphology after the embryo culture has been proposed (Non-Patent Document 5), there is no clear criterion in the method. Therefore, for the determination of a single embryo that is likely to lead to implantation and pregnancy, a biomarker capable of providing a better objective criterion by combination with the morphological evaluation has been demanded.

In recent years, a number of reports have been made in the research of biomarkers for infertility treatment. Patent Document 1 discloses MICA (MHC class I chain-related protein A) as a biomarker for noninvasive evaluation of in vitro fertilization. The biomarker has been proposed as a marker indicating the implantation failure rate, failure of in vitro fertilization, or abortion when the MICA level in a human sample exceeds a threshold. However, this biomarker does not indicate the quality of an actual ovum or embryo.

Patent Document 2 proposes soluble CD146 as a biomarker for preliminarily selecting an embryo suitable for intrauterine implantation. Although the document discloses a method of selecting an embryo suitable for transfer using as an index soluble CD146, which can be observed in an embryo culture, the method is not intended for human body fluids.

Patent Document 3 discloses genetic means for identifying pregnancy-competent oocytes. The document discloses an index based on assay evaluation of genes obtained from cumulus cells, which index enables viable pregnancy in transfer of the corresponding oocyte. However, no protein-level study has been carried out, and the means is not intended for human body fluids.

CD163, on which the present invention focuses attention, is known as a hemoglobin/haptoglobin scavenger receptor, and belongs to the scavenger receptor cysteine-rich superfamily. CD163 is a type I transmembrane protein expressed in monocytes or macrophages. In the course of the macrophage activation, soluble CD163 is generated by cleavage by metalloproteinase, and the soluble CD163 circulates in blood. Therefore circulating soluble CD163 has been reported as a marker for the macrophage activation, and recently also reported as a marker for predicting the therapeutic effect of nivolumab, which is a molecular-targeted drug aiming at the treatment of malignant melanoma or advanced-stage non-small-cell lung cancer (Patent Document 4).

Patent Document 5 discloses a kit for evaluating endometrial receptivity. Non-Patent Document 6 reports a study evaluating whether soluble CD163 and C-reactive protein can predict spontaneous preterm delivery in women with symptoms of preterm delivery. Non-Patent Document 7 reports a study exploring the predictive value of endometrial CD138 expression in the natural cycle preceding frozen embryo transfer in patients with normal endometrial dating and histopathologic features, who previously failed the transfer of two high-quality fresh embryos.

### DOCUMENTS OF THE ART

### [Patent Documents]

[Patent Document 1] WO 2008/084105
[Patent Document 2] WO 2016/170021
[Patent Document 3] WO 2011/060080
[Patent Document 4] WO 2018/003995
[Patent Document 5] CN 111 579 798 A

### [Non-patent Documents]

[Non-Patent Document 1] Japan Association of Obstetricians and Gynecologists ART Databook 2018
[Non-Patent Document 2] Japan Association of Obstetricians and Gynecologists, "Opinion on the Prevention of Multiple Pregnancy in Artificial Reproductive Technology", published in April, 2008
[Non-Patent Document 3] Atlas of The Human Oocyte & Early Conceptus, 2, 1991
[Non-Patent Document 4] Gardner DK, et al, Fertil. Steril., 73: 1155-1158, 2000
[Non-Patent Document 5] Pribenszky et al, Reprod. Biomed. Online, 35(5): 511-520, 2017
[Non-Patent Document 6] Vogel et al, BJOG: An International Journal of Obstetrics and Gynecology, 112(6): 737-742, 2005[Non-Patent Document 7] Fan Xiangxiu et al, Fertility and Sterility, 112(6): 1103-1111, 2019

### SUMMARY OF THE INVENTION

### PROBLEMS TO BE SOLVED BY THE INVENTION

The invention is as set out in the independent claims, further aspects of the invention are outlined in the dependent claims. Embodiments that do not fall within the scope of the claims do not describe part of the invention. An object of the present invention is to provide (a) use of soluble CD163 as a biomarker that enables identification of an embryo that leads to a high rate of implantation and pregnancy, that is, an embryo that leads to high fertility, from embryos obtained through embryo culture after in vitro fertilization in infertility treatment, (b) a determination method for determining fertility of an ovum or an embryo that develops from an ovum after fertilization, (c) use of a determination reagent or a determination kit using an antibody that specifically recognizes soluble CD163, and (d) use of an antibody that specifically recognizes soluble CD163 in determination of fertility.

### MEANS FOR SOLVING THE PROBLEMS

As a result of intensive study, the present inventors discovered that fertility of an ovum, or an embryo that develops from an ovum after fertilization, can be determined by measuring the concentration of soluble CD163 present in a body fluid such as follicular fluid collected at the same time as ova in the ovum pick up in in vitro fertilization, or serum immediately before the ovum pick up, thereby completing the present invention described below.

More specifically, the present invention is as follows.
[1] Use of soluble CD163 as a biomarker for determining fertility of an ovum, or an embryo that develops from an ovum after fertilization.
[2] A method of measuring the soluble CD163 concentration in a human body fluid in vitro, and determining fertility of an ovum, or an embryo that develops from an ovum after fertilization, based on the measured value.
[3] The method according to [2], wherein the ovum or the embryo that develops from an ovum after fertilization is determined to have high fertility in a case where the soluble CD163 concentration is higher than a standard value, the standard value being a soluble CD163 concentration in a body fluid of another human whose pregnancy did not establish after transfer of an embryo that develops from a human ovum after fertilization.
[4] The method according to [2] or [3], wherein the body fluid is follicular fluid, whole blood, serum, plasma, or urine.
[5] The method according to any one of [2] to [4], wherein the method of measuring the soluble CD163 concentration is an immunoassay.
[6] Use of a determination reagent or a determination kit for use in the method according to [5], wherein the reagent or kit comprises an antibody that specifically recognizes soluble CD163.
[7] Use of an antibody that specifically recognizes soluble CD163, in determination of fertility.
In aspects not forming part of the claimed invention, the present document discloses (i)-(iv) below:
(i) A biomarker for use in determining fertility of an ovum, or an embryo that develops from an ovum after fertilization, the biomarker comprising soluble CD163.
(ii) Use of an antibody that specifically recognizes soluble CD163, in the production of a reagent or a determination kit for determining fertility.
(iii) An antibody that specifically recognizes soluble CD163, for use in determination of fertility.
(iv) A method of treating infertility in a patient or a method of increasing establishment of pregnancy in in vitro fertilization, the method comprising the steps of:
   (i) measuring the soluble CD163 concentration in a body fluid collected from a patient;
   (ii) identifying, in a case where the measured value exceeds a predetermined standard value, an ovum collected from the patient on the same day as the collection of the sample, or an embryo that has developed from the ovum after fertilization, as having high fertility; and
   (iii) transferring an embryo that has developed from the identified ovum after fertilization, or the identified embryo, to the patient.

### EFFECT OF THE INVENTION

According to the present invention, selection of a suitable embryo can be aided based on an objective numerical value, so that evaluation and selection of an embryo by a physician can be greatly assisted by the invention.

### BRIEF DESCRIPTION OF THE DRAWINGS

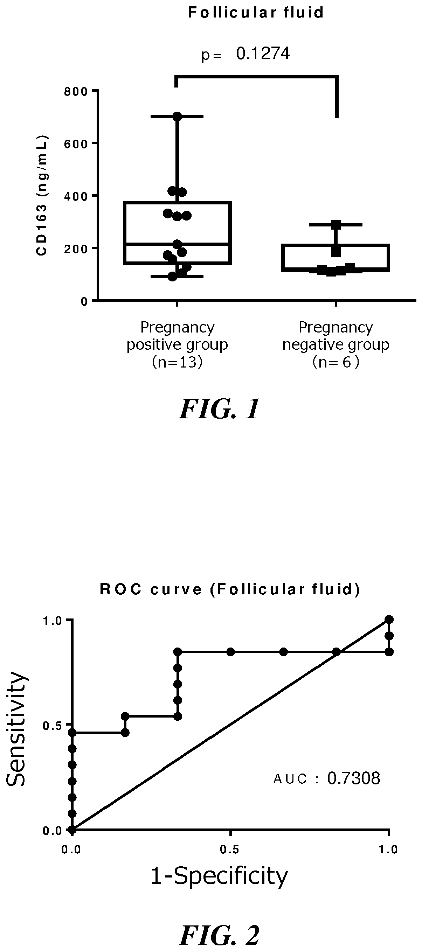

Fig. 1 is a box plot illustrating the result of comparison of measured values of the soluble CD163 level in follicular fluids using an anti-CD163 antibody, which comparison was made between follicular fluids that had contained an ovum with which pregnancy was established after embryo transfer and follicular fluids that had contained an ovum with which pregnancy was not established after embryo transfer. P=0.127 (Mann Whitney test).
Fig. 2 is a box plot illustrating the result of a receiver operating characteristic (ROC) curve analysis of soluble CD163 for follicular fluids that had contained an ovum with which pregnancy was established after embryo transfer and follicular fluids that had contained an ovum with which pregnancy was not established after embryo transfer.

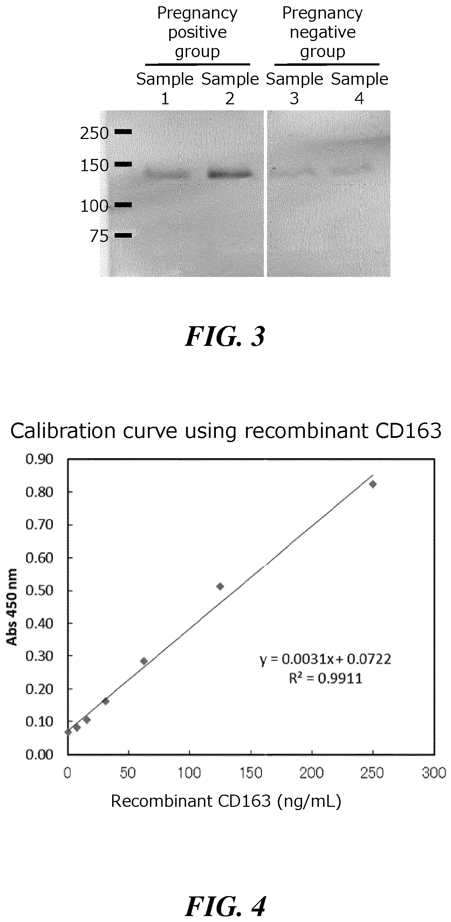

Fig. 3 is the result of Western blotting using an anti-CD163 antibody, which was carried out for follicular fluids that had contained an ovum with which pregnancy was established after embryo transfer and follicular fluids that had contained an ovum with which pregnancy was not established after embryo transfer. The pregnancy positive group clearly showed more intense bands than the pregnancy negative group.
Fig. 4 is a calibration curve prepared using recombinant CD163 based on the measurement system in Measurement Example 1.

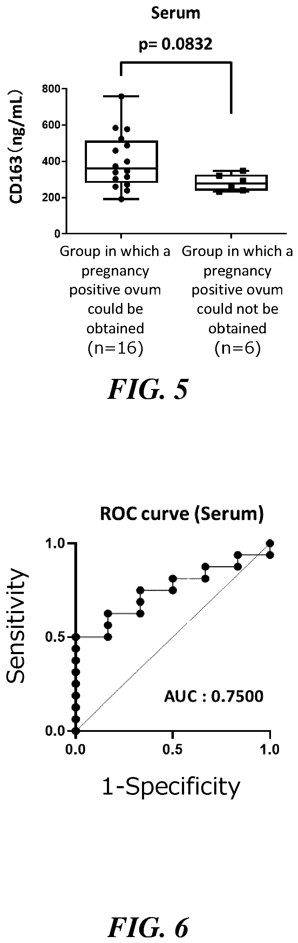

Fig. 5 is a box plot illustrating the result of comparison of measured values of the soluble CD163 level using an anti-CD163 antibody, which comparison was made between sera immediately before the ovum pick up in patients from whom an ovum that led to pregnancy after embryo transfer could be collected and sera immediately before the ovum pick up in patients from whom an ovum that led to pregnancy after embryo transfer could not be collected. P=0.0832 (Mann Whitney test).
Fig. 6 is a diagram illustrating the result of a receiver operating characteristic (ROC) curve analysis of soluble CD163 for sera immediately before the ovum pick up in patients from whom an ovum that led to pregnancy after embryo transfer could be collected and sera immediately before the ovum pick up in patients from whom an ovum that led to pregnancy after embryo transfer could not be collected.

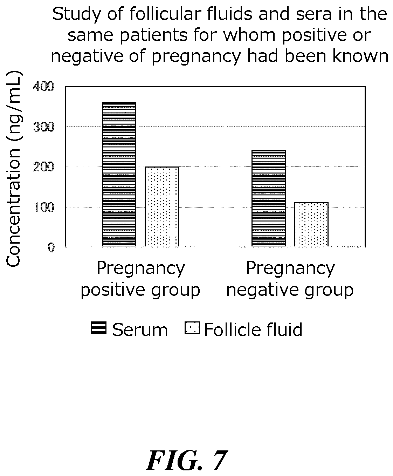

Fig. 7 is a diagram illustrating the result of comparison of medians of soluble CD163 using an anti-CD163 antibody, which comparison was made among sera immediately before the ovum pick up in patients from whom an ovum that led to pregnancy after embryo transfer could be collected, pregnancy positive follicular fluids of these patients, sera immediately before the ovum pick up in patients from whom an ovum that led to pregnancy after embryo transfer could not be collected, and pregnancy negative follicular fluids of these patients.

### MODE FOR CARRYING OUT THE INVENTION

A first mode of the present invention is the use of soluble CD163 as a biomarker for determining fertility of an ovum, or an embryo that develops from an ovum after fertilization, in infertility treatment. The biomarker comprises soluble CD163 present in a human body fluid.

CD163, which is a protein having a molecular weight of about 130 to 160 kDa, is a transmembrane protein expressed on monocytes and macrophages. After activation of macrophages by lipopolysaccharide (LPS) and the like, soluble CD163 is generated by cleavage from the membrane by metalloproteinase, and then released in the body.

CD163 includes four kinds of isoforms having different sequences mainly in the C-terminal portion (Uniprotkb protein isoforms: Q86VB7-1, 2, 3, 4; their amino acid sequences are shown in SEQ ID NOs:1 to 4, respectively). These four isoforms are cleaved at their specific sites to generate soluble CD163. As a result, identical soluble CD163 molecules are generated from CD163 isoforms 1, 2, and 3. However, a soluble CD163 molecule having a different sequence is generated from CD163 isoform 4. Both kinds of soluble CD163 have sequences corresponding to positions 42 to 578 and positions 580 to 1050 of SEQ ID NO:1. The biomarker may be one or both of the two kinds of soluble CD163.

As shown in Examples below, the soluble CD163 concentration (also referred to as "level") in the follicular fluid that had contained an ovum with which pregnancy was established after embryo transfer or in the serum immediately before the ovum pick up in the patient is significantly higher than that in the follicular fluid that had contained an ovum with which pregnancy was not established after embryo transfer or in the serum immediately before the ovum pick up in the patient. Therefore, soluble CD163 in the follicular fluid or in the serum immediately before the ovum pick up can be an index for determining fertility of an ovum, or an embryo that develops from an ovum after fertilization.

As such, the present invention provides the use of soluble CD163 as a biomarker for determining fertility of an ovum, or an embryo that develops from an ovum after fertilization.

A second mode of the present invention, based on such a discovery, is a method of measuring the soluble CD163 concentration in a human body fluid in vitro, and determining fertility of an ovum, or an embryo that develops from an ovum after fertilization, based on the measured value. This method enables selection of an ovum, or an embryo that develops from an ovum after fertilization, having high fertility. As a result, in addition to the conventional morphological evaluation of the blastocyst by microscopy, another basis for selecting an embryo suitable for transfer to the uterus is provided to increase the rate of establishment of pregnancy.

It should be noted that, while the method of the present invention includes the process to the determination of fertility, the method does not include the act of final determination of whether or not an embryo transfer should be carried out. A physician refers to the judgment result by the method of the present invention and the like to, for example, determine whether or not the transfer to the uterus should be carried out, select an embryo suitable for the transfer, or determine the course of infertility treatment.

The "fertility" herein means the level of the ability of an ovum, or an embryo that develops from an ovum after fertilization, to achieve the entire process from implantation to pregnancy. By selecting an embryo having high fertility before the implantation to the uterus, the rate of establishment of pregnancy can be increased.

The soluble CD163 level in a human sample can be investigated by measuring soluble CD163 protein or a fragment thereof in the sample. Since CD163 is a membrane-bound protein, it is present in the fragmented form, that is, soluble CD163, in a body fluid. The soluble CD163 protein or its fragment to be measured includes a protein or fragment that is present in a form in which the protein or fragment is bound to or associated with another protein or the like in the sample (for example, in a form in which it constitutes a complete or partially degraded soluble CD163 molecule in the sample). Accordingly, the term "soluble CD163" as used to represent the biomarker used in the present invention encompasses the full-length protein of soluble CD163 and a partial fragment thereof, and soluble CD163 protein and a partial fragment thereof in a form in which it is bound to or associated with another protein or protein fragment, present in a sample.

The soluble CD163 measured in the method of the present invention may be one or both of the two kinds of soluble CD163 described above.

The human sample is a human body fluid, and examples of the human body fluid that may be used include follicular fluid, blood, serum, plasma, and urine. Follicular fluid, blood, serum, or plasma is preferably used. Follicular fluid or serum is especially preferably used. The sample used in the method of the present invention means a sample collected, in other words, isolated, from a subject.

The human (subject) from whom the sample is collected is usually a female patient for whom in vitro fertilization is indicated in infertility treatment.

The collection of the sample from the subject is performed on the day of, preferably at the same time as, the ovum pick up which is usually carried out before ovulation. More specifically, the sample is collected about 10 to 14 days after the beginning of menstruation. In cases where the sample is follicular fluid, it is usually collected at the same time as the collection of an ovum (ovum pick up) from an ovary. In cases where the sample is blood, serum, plasma, urine, or the like, the sample is preferably collected immediately before the ovum pick up since, in such cases, the sample better reflects the conditions of the collected ova.

The timing of the measurement and determination in the method of the present invention is not limited as long as it is before the embryo transfer. It may be, for example, after the ovum pick up, after the fertilization, or after the cleavage has proceeded to the blastocyst. Usually, the measurement and determination are carried out by the time when a physician decides whether or not the embryo transfer should be carried out. Preferably, the result of morphological grading by a physician based on microscopic observation of the blastocyst, and the determination result by the method of the present invention, are obtained by the time of the decision of whether or not the transfer should be carried out.

The measurement and determination by the method of the present invention are preferably carried out in an early period after the ovum pick up, from the viewpoint of the efficiency of the infertility treatment. This is because the rate of pregnancy can be increased by allowing an ovum judged to have high fertility to proceed to the step of fertilization and the step of culture.

Follicular fluid is present in a mature ovarian follicle. The follicular fluid is collected together with an ovum at the time of ovum pick up using an ovum pick up needle from an ovarian follicle that has been maturated under an appropriately controlled induction of ovulation. Since the follicular fluid is discarded in ordinary infertility treatment, the determination by the present invention does not place any additional burden on the patient.

The method of measuring soluble CD163 in the human sample is not limited as long as the method is a quantitative measurement method such as immunoassay, liquid chromatography, electrophoresis, or mass spectrometry. Immunoassay may be preferably used in the present invention since it requires no large-scale apparatus, and can be simply operated.

Immunoassay per se is well known in the art. Based on the reaction mode, immunoassay can be divided into the sandwich method, competition method, agglutination method, Western blotting method, and the like. Based on the label, immunoassay can be divided into enzyme immunoassay, radioimmunoassay, fluorescence immunoassay, and the like. In the present invention, any immunoassay method capable of quantitative detection may be used. Preferred examples of such a method include, but are not limited to, sandwich methods such as sandwich ELISA.

The production method for the antibody used in the present invention is not limited. The antibody is typically a non-human animal polyclonal or monoclonal antibody prepared with a non-human animal such as a mouse or rabbit. Since the amino acid sequence of soluble CD163 and the base sequence encoding it are known as described above, a soluble-CD163 antibody that specifically recognizes a specific site of soluble CD163 may be prepared by a conventional hybridoma method or the like, and may then be used.

The antibody used in the present invention may be either a polyclonal antibody or a monoclonal antibody. An antiserum may be used as the polyclonal antibody. In the present disclosure, the term "polyclonal antibody" also encompasses an antiserum before purification. An antigen-binding fragment of the antibody may be used instead of the antibody. In the present description, the term "antibody" hereinafter encompasses an antigen-binding fragment of the antibody unless the context clearly indicates otherwise. All of the polyclonal antibody, monoclonal antibody, and antigen-binding fragment can be prepared by well-known conventional methods.

More specifically, a polyclonal antibody that specifically recognizes a specific site of soluble CD163 can be obtained by, for example, blending a plurality of kinds of monoclonal antibodies that specifically recognize the site. Alternatively, a non-human animal may be immunized using a polypeptide containing the site of soluble CD163 prepared by a well-known method such as chemical synthesis, or using soluble CD163 protein, or using a polynucleotide encoding the polypeptide or protein, or the like as an immunogen, together with an adjuvant when appropriate. By obtaining antiserum from blood collected from the animal, and carrying out purification, a polyclonal antibody (non-human-animal anti-soluble-CD163 polyclonal antibody) can be obtained from the antiserum. Normally, in order to increase the antibody titer in the immunized animal, the immunization is carried out a plurality of times for several weeks. The antibody in the antiserum can be purified by, for example, fractionation by ammonium sulfate precipitation or anion chromatography, or affinity column purification.

An example of well-known methods for preparation of a monoclonal antibody is the hybridoma method. More specifically, for example, antibody-producing cells such as spleen cells or lymphocytes may be collected from a non-human animal immunized as described above, and the collected cells may be fused with myeloma cells to prepare hybridomas, followed by selecting a hybridoma producing an antibody that binds to a specific site of soluble CD163, and then growing the hybridoma. From the resulting culture supernatant, a monoclonal antibody that specifically recognizes the specific site of non-human-animal soluble-CD163 can be obtained.

The "antigen-binding fragment" means an antibody fragment having the binding capacity to the corresponding antigen of the antibody (antigen-antibody reactivity), such as the Fab fragment or F(ab')₂ fragment of immunoglobulin. It is well known that such an antigen-binding fragment can also be used in immunoassays, and the fragment is useful similarly to its original antibody. Fab fragments and F(ab')₂ fragments can be obtained, as is well known, by treating antibodies with a protease such as papain or pepsin. The antigen-binding fragment is not limited to a Fab fragment or F(ab')₂ fragment, and may be any fragment having the binding capacity to its corresponding antigen. It may also be a fragment prepared by a genetic engineering method. For example, an antibody prepared by expressing a single chain fragment of variable region (scFv) in *E. coli* by a genetic engineering method may be used. A method of preparing an scFv is also well known. An scFv can be prepared by extracting mRNA from a hybridoma prepared as described above, preparing single-stranded cDNA therefrom, performing PCR using primers specific to the H-chain and L-chain of immunoglobulin to amplify the immunoglobulin H-chain gene and L-chain gene, linking these genes together via a linker, introducing the resulting product, after adding appropriate restriction sites thereto, to a plasmid vector, transforming *E. coli* with the resulting plasmid vector, and then recovering scFv from the transformed *E. coli.* Such an scFv is also included in the "antigen-binding fragment".

Immunoassay per se is a well-known technique. Briefly, for example, in a sandwich immunoassay, an antibody that binds to soluble CD163 is immobilized on a solid phase (immobilized antibody), and then reacted with a sample, followed by washing if necessary. Thereafter, the reaction product is reacted with a labeled antibody prepared by adding a label to an antibody that binds to soluble CD163 at a site which is the same as or different from the site to which the immobilized antibody binds. After washing, the labeled antibody bound to the solid phase is measured.

The measurement of the labeled antibody can be carried out by measuring a signal from the labeling substance. The method for measuring the signal is appropriately selected depending on the type of the labeling substance. For example, in cases of an enzyme label, a substrate compatible with the enzyme, such as a chromogenic substrate, fluorescent substrate, or luminescent substrate, may be reacted with the enzyme, and a signal generated as a result such as coloring or luminescence may be measured using an appropriate apparatus such as an absorptiometer or luminometer, to determine the enzyme activity to thereby measure the target of measurement. For example, in cases where ALP is used as the labeling substance, a luminescent substrate such as disodium 3-(4-methoxyspiro(1,2-dioxetane-3,2'-tricyclo[3.3.1.13,7]decan)-4-yl)phenylphosphate (trade name, AMPPD) may be used. In the labeled antibody, the labeling substance may be directly bound to the antibody. Alternatively, the labeling substance may be indirectly bound by binding a specific binding molecule such as biotin or hapten to the antibody and reacting the resulting product with a partner (for example, streptavidin or an anti-hapten antibody) of the specific binding molecule, which partner has a labeling substance bound thereto. Soluble CD163 in a sample can be quantified by preliminarily performing immunoassay of standard samples containing soluble CD163 at various known concentrations using an anti-soluble-CD163 antibody or an antigen-binding fragment thereof, and plotting the correlation between the amount of signal from the label and the soluble CD163 concentration in each standard sample to prepare a calibration curve, followed by carrying out the same operation for the sample whose soluble CD163 concentration is unknown to measure the amount of signal from the label, and then applying the measured value to the calibration curve.

Whether or not the soluble CD163 level in a body fluid is high can be determined by comparison with a threshold that is a standard value determined based on, for example, the soluble CD163 level in another body fluid that had originally contained the ovum of an embryo with which pregnancy was not established. The standard value may be, for example, the median of the soluble CD163 concentrations in a large number of follicular fluids that had originally contained the ova of embryos with which pregnancy was not established. In cases where the measured value of the soluble CD163 level in each follicular fluid obtained by ovum pick up as a test sample is higher than the standard value, it can be determined that the fertility is high, in other words, the quality of the ovum is high enough to lead to implantation and pregnancy. The threshold may be set for each age group (for example, younger than 30 years old, in their 30s, in their 40s, or in their 50s).

In the method of the present invention, the ovum or embryo whose fertility is to be determined is an ovum collected on the day of, or at the time of, the collection of the sample to be subjected to the measurement, or an embryo that has developed from the ovum after fertilization.

In the method of the present invention, the embryo whose fertility can be determined may be either a cleavage-stage embryo or a blastocyst. The embryo is preferably a blastocyst.

In an aspect not forming part of the claimed invention, the method of determining the fertility may be applied to a method of infertility treatment. More specifically, a method of treating infertility in a patient or a method of increasing establishment of pregnancy in in vitro fertilization, may comprise the steps of:
(i) measuring the soluble CD163 concentration in a body fluid collected from the patient;
(ii) identifying, in a case where the measured value exceeds a predetermined standard value, an ovum collected from the patient on the same day as the collection of the sample, or an embryo that has developed from the ovum after fertilization, as having high fertility; and
(iii) transferring an embryo that has developed from the identified ovum after fertilization, or the identified embryo, to the patient.

A third mode of the present invention relates to the use of a determination reagent for the method of determining fertility according to the second mode above, wherein the reagent comprises an antibody that specifically recognizes soluble CD163.

The determination reagent may be composed only of an antibody or an antigen-binding fragment that specifically recognizes soluble CD163, or may also contain another component useful for stabilization or the like of the antibody or the antigen-binding fragment thereof. The antibody or the antigen-binding fragment thereof may be in a form in which a labeling substance or a specific binding molecule such as biotin is bound thereto, or in a form in which the antibody or antigen-binding fragment is immobilized on a solid phase such as a plate or particle.

A fourth mode of the present invention may be the use of a kit for determining fertility, comprising the determination reagent used in the present invention which reagent comprises an antibody that specifically recognizes soluble CD163. The kit is an immunoassay kit, and may also contain another reagent or the like required for immunoassay. Other reagents required for immunoassay are well known. For example, in addition to the determination reagent described above, the kit may comprise a sample diluent, a washing liquid, and, when the labeling substance used for the labeled antibody is an enzyme, a substrate liquid for the enzyme, and the like. Further, the kit usually includes instructions for use.

Another aspect of this mode, not forming part of the claimed invention, is use of an antibody that specifically recognizes soluble CD163, in the production of a reagent for determining fertility.

Still another aspect of the present invention is use of an antibody that specifically recognizes soluble CD163, in determination of fertility.

Still another aspect not forming part of the claimed invention is an antibody that specifically recognizes soluble CD163, for use in determination of fertility.

### EXAMPLES

The present invention is illustrated below in more detail by way of Examples.

### <Measurement Example 1> Measurement of Soluble CD163 in Follicular Fluid by ELISA Using Anti-CD163 Antibody

As follicular fluid samples that had contained an ovum for which positive or negative of pregnancy had been found, those that could be collected at the same time as collection of ova from patients for whom ovulation was appropriately induced were employed. The follicular fluids were stored at -80°C after the collection. By checking culture records of the collected ova and whether or not pregnancy was established in the patients, the follicular fluids were divided into a pregnancy positive group and a pregnancy negative group, which included 13 follicular fluid samples for which establishment of pregnancy was found and 6 follicular fluid samples for which pregnancy was not established.

A purchased anti-CD163 polyclonal antibody (R&D Systems) was used. Since this antibody is an antibody prepared by immunization with a sequence corresponding to Gly46-Ser1050 of SEQ ID NO:1, soluble CD163 can be measured therewith. This antibody was diluted in carbonate buffer (pH 9.8) at 50 ng/well, and immobilized on a MaxiSorp 96-well plate (manufactured by Nunc). The reaction was allowed to proceed at 4°C overnight, and then the wells were washed three times with TBS-T (Tris-buffered saline supplemented with 0.05% Tween 20), followed by adding TBS solution supplemented with 3% bovine serum albumin (BSA) to the wells at 200 µL/well, and leaving the plate to stand at room temperature for 2 hours.

After washing the wells three times with TBS-T, the 13 samples in the pregnancy positive follicular fluid group or the 6 samples in the pregnancy negative follicular fluid group were diluted 4-fold with TBS-T solution supplemented with 1% bovine serum albumin, and added to the wells at 40 µL/well, followed by leaving the plate to stand at room temperature for 1 hour.

After washing the wells three times with TBS-T, a purchased anti-CD163 monoclonal antibody (R&D Systems) was diluted to 1.0 µg/mL with TBS-T solution supplemented with 1% bovine serum albumin, and added to the wells at 40 µL/well, followed by leaving the plate to stand at room temperature for 1 hour. After washing the wells three times with TBS-T, a horseradish peroxidase (HRP)-labeled anti-mouse IgG (manufactured by SIGMA) solution diluted 20,000-fold with TBS-T solution supplemented with 1% bovine serum albumin was added to the wells at 40 µL/well, followed by leaving the plate to stand at room temperature for 1 hour. After washing the wells four times with TBS-T, TMB Microwell Peroxidase Substrate (manufactured by KPL) was added thereto, and then the reaction was stopped with 1 mol/L phosphate solution, followed by measurement of the absorbance at 450 nm using an absorbance measurement plate reader. The measurement system in Measurement Example 1 enables measurement of soluble CD163 as described in Reference Example 1.

The results are shown in Fig. 1. Compared to the pregnancy negative group, the pregnancy positive group clearly showed higher values of soluble CD163 in the follicular fluids. While the median in the pregnancy negative group was 119.9 ng/mL, the median in the pregnancy positive group was 213.5 ng/mL.

Table 1 and Fig. 2 show results of evaluation, by ROC analysis, of the capacity of soluble CD163 in follicular fluid to detect establishment of pregnancy. The results suggested a relationship between the soluble CD163 concentration in follicular fluid, which is an independent variable, and the outcome, that is, the presence or absence of establishment of pregnancy, which is a dichotomous variable. The area under the ROC curve (AUC: area under the curve) was 0.73.

**[Table 1]**

| Area under the ROC curve | | |
|---|---|---|
| Area | | 0.73 |
| P-value | | 0.1274 |

| Number of samples | | |
|---|---|---|
| | Pregnancy positive samples | 13 |
| | Pregnancy negative samples | 6 |

### <Reference Example 1>

As a standard sample for a calibration curve, Recombinant Human CD163 Protein (a recombinant CD163), manufactured by R&D was used. The recombinant CD163 has the sequence corresponding to Gly46-Ser1050 of SEQ ID NO:1, and was prepared using mouse melanoma. Soluble CD163 has this sequence. The recombinant CD163 was conditioned with TBS-T solution supplemented with 1% bovine serum albumin, and measured by the measurement system in Measurement Example 1, to prepare a calibration curve. The result is shown in Fig. 4.

### <Measurement Example 2> Measurement of Soluble CD163 by Western Blotting Using Anti-CD163 Antibody

As follicular fluid samples that had contained an ovum for which pregnancy positive or negative of pregnancy had been found, follicular fluids that could be collected at the same time as collection of ova from patients for whom ovulation was induced were employed. The follicular fluids were stored at -80°C after the collection. By checking culture records of the collected ova and whether or not pregnancy was established in the patients, the follicular fluids were divided into a pregnancy positive group and a pregnancy negative group, which included 2 follicular fluid samples for which establishment of pregnancy was found and 2 follicular fluid samples for which pregnancy was not established.

The subject follicular fluids were pretreated using High Select (registered trademark) Top14 Abundant Protein Depletion Mini Spin Columns (manufactured by Thermo) according to a protocol. The protein concentration of the resulting eluate was measured, and the eluate was applied to 5-20% gradient gel at 10 µg/Lane, followed by performing SDS-PAGE. After the electrophoresis, protein was transferred to a PVDF membrane using a blotting apparatus. The PVDF membrane to which the protein was transferred was subjected to blocking treatment at room temperature for 1 hour using TBST buffer supplemented with 5% skim milk. After the blocking operation, the PVDF membrane was conditioned in TBST buffer supplemented with 5% skim milk, containing 1.0 µg/mL anti-CD163 polyclonal antibody (R&D Systems; the same antibody as used in Measurement Example 1), and the reaction was allowed to proceed at room temperature for 1 hour. Thereafter, the PVDF membrane was washed three times with TBST buffer, and conditioned in a horseradish peroxidase (HRP)-labeled anti-goat IgG (manufactured by SIGMA) solution diluted 2000-fold with TBST buffer supplemented with 5% skim milk, followed by allowing the reaction to proceed at room temperature for 1 hour. Thereafter, the membrane was washed three times with TBST buffer, and luminescence was generated by the ECL Prime Western Blotting Detection Reagent (GE Healthcare), followed by detection and observation using a CCD camera. Analysis of the signal of each detected band was carried out using Multi Gauge (Fujifilm).

The results are shown in Fig. 3. Clearly more intense bands could be detected for the follicular fluids in the pregnancy positive group compared to those in the pregnancy negative group.

The results of analysis of the signals are shown in Table 2. The follicular fluids in the pregnancy positive group showed clearly higher signals than those in the pregnancy negative group.

**[Table 2]**

| | Pregnancy positive group | | Pregnancy negative group | |
|---|---|---|---|---|
| | Sample 1 | Sample 2 | Sample 3 | Sample 4 |
| Intensity | 34158.41 | 55757.41 | 9935.41 | 9721.41 |

### <Measurement Example 3> Measurement of Soluble CD163 in Sera Collected Immediately before ovum Collection, by ELISA Using Anti-CD163 Antibody

As patient sera, those collected immediately before ovum pick up from patients for whom ovulation was appropriately induced were used. The sera were stored at -80°C after the collection. After performing in vitro fertilization using the collected ova, fertilized ova were transferred, and whether or not pregnancy was established in the patients was checked. The patients were divided into a group in which an ovum with which pregnancy was established could be collected and a group in which such an ovum could not be collected. Sixteen serum samples from patients from whom a pregnancy positive ovum could be collected, and 6 serum samples from patients from whom such an ovum could not be collected, were used.

Soluble CD163 in each sample was measured in the same manner as in Measurement Example 1.

The results are shown in Fig. 5. Compared to the group in which pregnancy positive ova could not be collected, the group in which pregnancy positive ova could be collected clearly showed higher values of soluble CD163 in the sera. While the median in the group in which pregnancy positive ova could not be collected was 278.0 ng/mL, the median in the pregnancy positive group was 360.2 ng/mL.

Table 3 and Fig. 6 show results of evaluation, by ROC analysis, of the capacity of soluble CD163 in serum to detect establishment of pregnancy. The results suggested a relationship between the soluble CD163 concentration in serum, which is an independent variable, and the outcome, that is, the presence or absence of establishment of pregnancy, which is a dichotomous variable. The area under the ROC curve was 0.75.

**[Table 3]**

| Area under the ROC curve | | |
|---|---|---|
| Area | | 0.75 |
| P-value | | 0.0832 |

| Number of samples | | |
|---|---|---|
| | Collection of an ovum that led to establishment of pregnancy | 16 |
| | Collection of an Ovum that led to no-establishment of pregnancy | 6 |

### <Measurement Example 4> Measurement of Soluble CD163 in Sera Collected Immediately before Ovum Collection and in Follicular Fluids, by ELISA Using Anti-CD163 Antibody

As follicular fluid samples that had contained an ovum for which pregnancy positive or negative of pregnancy had been found, those that could be collected at the same time as collection of ova from patients for whom ovulation was appropriately induced were employed. The follicular fluids were stored at -80°C after the collection. As patient sera, those collected immediately before ovum pick up from patients for whom ovulation was appropriately induced were used. The sera were stored at -80°C after the collection. After performing in vitro fertilization using the collected ova, fertilized ova were transferred to the patients, and whether or not pregnancy was established as a result was checked. The patients were divided into a group in which an ovum with which pregnancy was established could be collected and a group in which such an ovum could not be collected. Twelve serum samples from patients for whom establishment of pregnancy was found, 12 pregnancy positive follicular fluid samples from these patients, 3 serum samples from patients from whom a pregnancy positive ovum could not be collected, and 3 pregnancy negative follicular fluid samples from these patients, were used.

Soluble CD163 in each sample was measured in the same manner as in Measurement Example 1.

The results are shown in Fig. 7. The sera from the patients from whom the ova for which establishment of pregnancy was found could be collected, and the pregnancy success follicular fluids of these patients, clearly showed higher values of soluble CD163 in the follicular fluids and the sera compared to those in the group in which pregnancy positive ova could not be collected. In the group in which pregnancy positive ova could not be collected, the serum median was 240.5 ng/mL, and the follicular fluid median was 112.0 ng/mL. In contrast, in the pregnancy positive group, the serum median was 360.2 ng/mL, and the follicular fluid median was 198.4 ng/mL.

## Claims

1. In vitro use of soluble CD163 as a biomarker for determining fertility of an ovum, or an embryo that develops from an ovum after fertilization.

2. A method of measuring the soluble CD163 concentration in a human body fluid in vitro, and determining fertility of an ovum, or an embryo that develops from an ovum after fertilization, based on the measured value.

3. The method according to claim 2, wherein the ovum or the embryo that develops from an ovum after fertilization is determined to have high fertility in a case where the soluble CD163 concentration is higher than a standard value, the standard value being a soluble CD163 concentration in a body fluid of another human whose pregnancy did not establish after transfer of an embryo that develops from a human ovum after fertilization.

4. The method according to claim 2 or 3, wherein the body fluid is follicular fluid, whole blood, serum, plasma, or urine.

5. The method according to any one of claims 2 to 4, wherein the method of measuring the soluble CD163 concentration is an immunoassay.

6. Use of determination reagent or a determination kit for the method according to claim 5, wherein the reagent or kit comprises an antibody that specifically recognizes soluble CD163.

7. In vitro use of an antibody that specifically recognizes soluble CD163, in determination of fertility.

## Patentansprüche

1. In-vitro-Verwendung von löslichem CD163 als ein Biomarker zur Bestimmung der Fruchtbarkeit einer Eizelle oder eines Embryos, der sich nach der Befruchtung aus einer Eizelle entwickelt.

2. Verfahren zur In-vitro-Messung der Konzentration an dem löslichen CD163 in einer menschlichen Körperflüssigkeit und Bestimmung der Fruchtbarkeit einer Eizelle oder eines Embryos, der sich nach der Befruchtung aus einer Eizelle entwickelt, auf der Grundlage des gemessenen Werts.

3. Verfahren nach Anspruch 2, wobei in einem Fall, in dem die Konzentration an löslichem CD163 höher ist als ein Standardwert, bestimmt wird, dass die Eizelle oder der Embryo, der sich nach der Befruchtung aus einer Eizelle entwickelt, eine hohe Fruchtbarkeit aufweist, wobei der Standardwert eine Konzentration an löslichem CD163 in einer Körperflüssigkeit eines anderen Menschen ist, dessen Schwangerschaft nicht nach dem Transfer eines Embryos festgestellt wurde, der sich aus einer menschlichen Eizelle nach der Befruchtung entwickelt.

4. Verfahren nach Anspruch 2 oder 3, wobei die Körperflüssigkeit Follikelflüssigkeit, Vollblut, Serum, Plasma oder Urin ist.

5. Verfahren nach einem der Ansprüche 2 bis 4, wobei das Verfahren zur Messung der Konzentration an löslichem CD163 ein Immunassay ist.

6. Verwendung von Bestimmungsreagens oder eines Bestimmungs-Kits für das Verfahren nach Anspruch 5, wobei das Reagens oder Kit einen Antikörper umfasst, der spezifisch lösliches CD163 erkennt.

7. In-vitro-Verwendung eines Antikörpers, der spezifisch lösliches CD163 erkennt, bei der Bestimmung der Fruchtbarkeit.

## Revendications

1. Utilisation in vitro de CD163 soluble en tant que biomarqueur pour déterminer la fertilité d'un ovule, ou d'un embryon qui se développe à partir d'un ovule après fertilisation.

2. Procédé de mesure de la concentration de CD163 soluble dans un fluide corporel humain in vitro, et de détermination de la fertilité d'un ovule, ou d'un embryon qui se développe à partir d'un ovule après fertilisation, basé sur la valeur mesurée.

3. Procédé selon la revendication 2, dans lequel l'ovule ou l'embryon qui se développe à partir d'un ovule après fertilisation est déterminé avoir une fertilité élevée dans le cas où la concentration de CD163 soluble est supérieure à une valeur standard, la valeur standard étant la concentration de CD163 soluble dans un fluide corporel d'un autre humain dont la gestation ne s'est pas établie après transfert d'un embryon qui se développe à partir d'un ovule humain après fertilisation.

4. Procédé selon la revendication 2 ou 3, dans lequel le fluide corporel est du fluide folliculaire, du sang entier, du sérum, du plasma, ou de l'urine.

5. Procédé selon l'une quelconque des revendications 2 à 4, dans lequel le procédé de mesure de la concentration de CD163 soluble est un immunodosage.

6. Utilisation d'un réactif de détermination ou d'une trousse de détermination pour le procédé selon la revendication 5, dans laquelle le réactif ou la trousse comprend un anticorps qui reconnaît spécifiquement le CD163 soluble.

7. Utilisation in vitro d'un anticorps qui reconnaît spécifiquement le CD163 soluble, dans une détermination de fertilité.
